# EUROPEAN PATENT APPLICATION

(11) **EP 4 382 153 A1**
(43) Date of publication of application: **12.06.2024**
(21) Application number: 22853016.8
(22) Date of filing: 01.08.2022
(51) Int. Cl.: A61M 11/00

(54) **NASAL SPRAY/INJECTION NOZZLE**

(30) Priority: 02.08.2021 JP 2021126673
(71) Applicant: Toko Yakuhin Kogyo Co., Ltd., Osaka-shi, Osaka 530-0022 (JP)
(72) Inventor: KAMISHITA, Taizou, Osaka-shi, Osaka 530-0022 (JP); MIYAZAKI, Takashi, Osaka-shi, Osaka 530-0022 (JP)
(74) Representative: Hoffmann Eitle
(86) International application number: PCT/JP2022/029525
(87) International publication number: WO 2023/013597

(57) **Abstract**

A nasal spray/injection nozzle according to one embodiment of the present invention comprises a pair of sub-nozzles. Each of the sub-nozzles has an ejection hole formed at the leading end. The sub-nozzles are coupled to each other through a joining section. The separation distance between the axis of one of the sub-nozzles and the axis of the other sub-nozzle increases from the joining section toward the respective leading ends.

## Description

### TECHNICAL FIELD

The present invention relates to a nasal spray/spout nozzle and a nasal spray/spout device for administering a medicament into a nasal cavity.

### BACKGROUND ART

Conventionally, an administration of a medicament has been widely performed in such a way that a spray/spout device is charged with a liquid agent, followed by spraying or spouting of the liquid agent onto a mucosa or skin of a body lumen/cavity or body surface by means of the device. In particular, the medicament for an intranasal administration has been widely available.

### PRIOR ART DOCUMENT

### PATENT DOCUMENTS

Patent Document 1: JP3371222
Patent Document 2: JP3052028
Patent Document 3: JP5959269
Patent Document 4: JP-A-2007-105365

### SUMMARY OF THE INVENTION

### PROBLEMS TO BE SOLVED BY THE INVENTION

As a nasal spray/spout device, for example, a nasal spray/spout device capable of simultaneously spraying/spouting a medicament into the two nasal cavities may be used from the viewpoint of simultaneously completing the administration of the medicament to the two nasal cavities by one operation (Patent Documents 1 to 4).

The inventors of the present invention have found that there is still room for further improving the nasal spray/spout device in terms of a medicament delivery to an intended region. More specifically, it has been found by the inventors that a medicament spray/spout by use of the nasal spray/spout device that can spray/spout a medicament into the two nasal cavities can further improve the medicament delivery with respect to a target site located in the nasal cavity.

The present disclosure has been made in view of such problems. That is, a main object of the present invention is to provide a nasal spray/spout nozzle capable of further improving the medicament delivery onto the target site located in the nasal cavity.

### SOLUTIONS TO THE PROBLEMS

The inventors of this application have addressed the matters described above, from a novel standpoint rather than a continual standpoint of the conventional art. As a result, the inventors of this application have created a nasal spray/spout nozzle that is capable of attaining the main object described above.

The present invention provides a nasal spray/spout nozzle comprising a pair of sub nozzles, wherein
a tip of each of the sub nozzles is provided with a jetting port, and the sub nozzles are connected to each other via a connecting portion, and
a separation distance between an axis of one of the sub nozzles and an axis of the other sub nozzle is increased from the connecting portion toward the tip.

### EFFECTS OF THE INVENTION

The nasal spray/spout nozzle according to the present invention can improve the medicament delivery with respect to the target site located in the nasal cavity.

### BRIEF DESCRIPTION OF THE DRAWINGS

Figs. 1(A) to 1(C) are schematic views illustrating one embodiment of a nasal spray/spout nozzle according to the present invention (Fig. 1(A): perspective view, Fig. 1(B): side view, and Fig. 1(C): top view).
Fig. 2 is a schematic view illustrating one embodiment of the nasal spray/spout nozzle according to the present invention, showing a separation distance of an outer side surface of a sub nozzle.
Figs. 3(A) to 3(C) are schematic views illustrating one embodiment of the nasal spray/spout nozzle according to the present invention (Fig. 3(A): perspective view, Fig. 3(B): side view, and Fig. 3(C): top view).
Fig. 4 is a schematic view illustrating an angle θ formed by an axis of a sub nozzle of one embodiment of the nasal spray/spout nozzle according to the present invention and a line symmetry axis of the nasal spray/spout nozzle.
Figs. 5(A) to 5(C) are schematic views illustrating one embodiment of the nasal spray/spout nozzle according to the present invention (Fig. 5(A): perspective view, Fig. 5(B): side view, and Fig. 5(C): top view).
Figs. 6(A) to 6(C) are schematic views illustrating one embodiment of the nasal spray/spout nozzle according to the present invention (Fig. 6(A): perspective view, Fig. 6(B): side view, and Fig. 6(C): top view).
Figs. 7(A) to 7(C) are schematic views illustrating one embodiment of the nasal spray/spout nozzle according to the present invention (Fig. 7(A): perspective view, Fig. 7(B): side view, and Fig. 7(C): top view).
Figs. 8(A) to 8(C) are schematic views illustrating one embodiment of the nasal spray/spout nozzle according to the present invention (Fig. 8(A): perspective view, Fig. 8(B): side view, and Fig. 8(C): top view).
Figs. 9(A) to 9(C) are schematic views illustrating one embodiment of the nasal spray/spout nozzle according to the present invention (Fig. 9(A): perspective view, Fig. 9(B): side view, and Fig. 9(C): top view).
Figs. 10(A) to 10(C) are schematic views illustrating one embodiment of the nasal spray/spout nozzle according to the present invention (Fig. 10(A): perspective view, Fig. 10(B): side view, and Fig. 10(C): top view).
Fig. 11 is a schematic view illustrating a state in which the axis of each sub nozzle of the nasal spray/spout nozzle according to the present invention is increased outward.
Figs. 12(A) to 12(C) are schematic views illustrating one embodiment of the nasal spray/spout device according to the present invention (Fig. 12(A): perspective view, Fig. 12(B): side view, and Fig. 12(C): top view).
Fig. 13 is an exploded perspective view illustrating one embodiment of the nasal spray/spout device according to the present invention.

### DETAILED DESCRIPTION

Hereinafter, embodiments of the present invention will be described with reference to the accompanying drawings. The embodiments described below are intended to embody a basic technical concept of the present invention, and do not limit the present invention only to such embodiments unless otherwise specified.

In the drawings, some members having the same function as each other may be denoted by the same reference numeral. For suitable explanation of the main points of the present invention or easier understanding of the present invention, the embodiments may be separately described or shown, but a partial replacement or combination of the embodiments with each other is possible in terms of their configuration/structure. In the following embodiments, descriptions about matters that are common to those already described will be omitted, and in this case only difference(s) will be described. In particular, similar mechanisms and/or effects due to the similar configurations/structures will be omitted so as to avoid a duplicate explanation in each embodiment. The sizes, positional relationships, and the like of the member/portions illustrated in the drawings may be exaggerated for an improved clarity of explanation.

In the following description of the embodiments, terms regarding the directions (e.g., an "up-down direction", a "horizontal direction (left-right direction)", a "drawing-depth direction (perspective direction)", a "proximal side", a "distal side", and the like) are conveniently used for ease of understanding, and thus these terms are just for purpose of explaining the present invention so that the present invention is not adversely limited to them. In the accompanying drawings, similar portions/parts/members are illustrated by use of the same or similar reference numerals.

The "up-down direction", the "horizontal direction (left-right direction)", and the "drawing-depth direction (perspective direction)" as used herein correspond to those of drawings, and thus they correspond to an up-down direction, a horizontal direction, and a drawing-depth direction (perspective direction) in each of the drawings, respectively. For example, as for the "up-down direction", a downward direction in the verticality (i.e., a direction in which gravity acts) can refer to the "downward direction"/"lower", and also a direction opposite to the gravity-acting direction can refer to the "upward direction"/"upper".

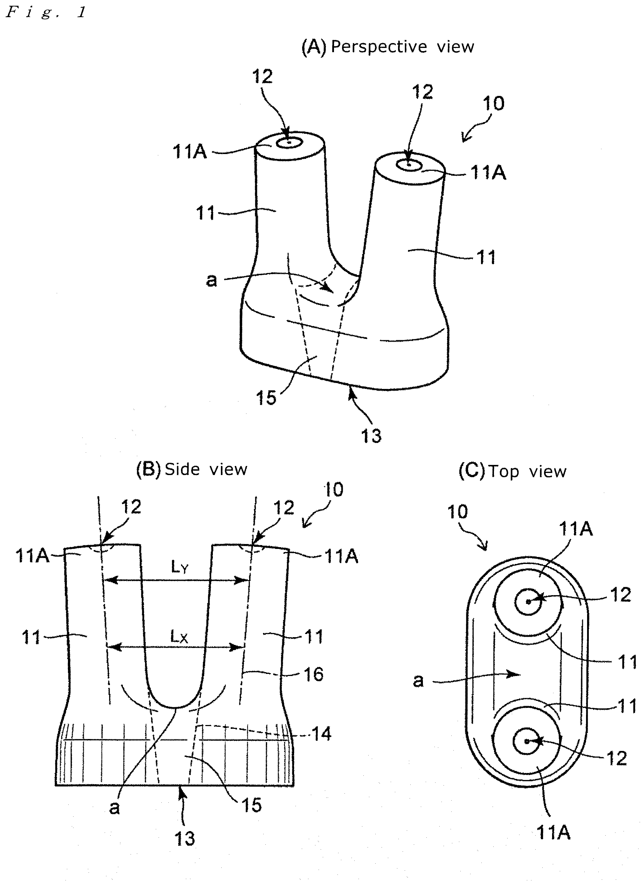

The term "side view" as used herein is based on a form/appearance when viewed in a direction that is approximately a perpendicular to the longitudinal direction of a sub nozzle. For example, the "side view" is based on forms/appearances as illustrated in Fig. 1(B) as well as other drawings. For example, the term "side view" is based on a form/appearance when the sub nozzle is viewed from a direction approximately equal to the horizontal direction among directions approximately perpendicular to the axial direction of the nozzle.

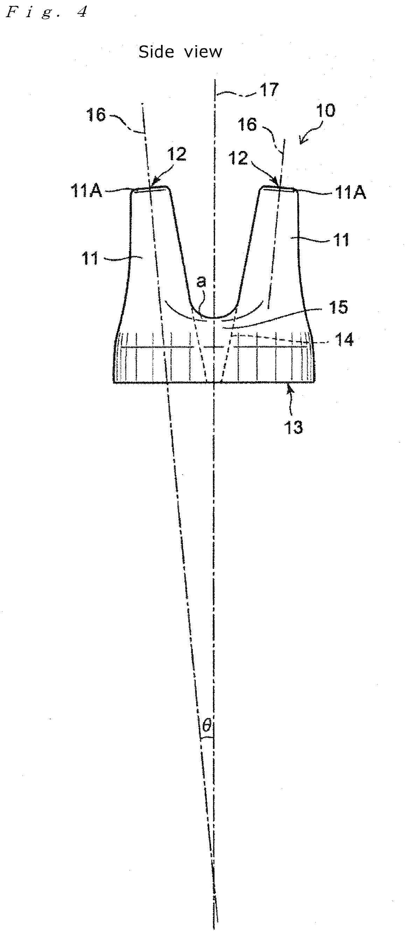

The term "top view" as used herein is based on a form/appearance when viewed from the side of a nozzle jetting port of the nasal spray/spout nozzle. For example, the "top plan view" is based on forms/appearances as illustrated in Fig. 1(C) as well as other drawings. Specifically, "top view" is based on the form/appearance when the nasal spray/spout nozzle (for example, the nozzle jetting port side thereof or a crotch portion of the nasal spray/spout nozzle) is viewed from the outside along the line symmetry axis direction of the nasal spray/spout nozzle (that is, the line symmetry axis 17 in Fig. 4).

The term "approximately" as used herein refers to a state/form/shape that is close to an exact state/form/shape, or close to a complete state/form/shape as a whole. For example, the phrase "approximately an elliptic shape" is not necessarily limited to a shape recognized by those skilled in the art as an ellipse in a strict or exact sense, but can include shapes that are each recognizable by those skilled in the art as an ellipse in a non-strict or non-exact sense. For example, even in a case where a part of the contour of a certain shape constitutes the contour of another shape, the certain shape having such contour can also be regarded as an ellipse as long as it can be recognized as an elliptical shape as a whole by those skilled in the art.

### [Features of Nasal Spray/Spout Nozzle of the Present Invention]

Hereinafter, the features of a nasal spray/spout device according to one embodiment of the present invention will be described.

A nasal spray/spout nozzle according to one embodiment of the present invention is a nasal spray/spout nozzle including a pair of sub nozzles, wherein a jetting port is provided at a tip of each of the sub nozzles, and the sub nozzles are connected to each other via a connecting portion. In particular, the separation distance between the axis of one sub nozzle and the axis of the other sub nozzle is increased from the connecting portion toward the tips.

Before the features of the present invention are described, the definition of terms will be described. The "nozzle" as used herein is like what can define an orientation of a liquid substance, preferably what can define a flow direction of a medicament (e.g., a liquid agent or a gel agent), and thus is like what can jet (spray, discharge, or spout) the liquid substance such as the medicament from a jetting port provided at the tip thereof. To achieve the above-described function, the nozzle preferably includes a hollow portion communicating with the jetting port therein (the hollow portion can also communicate with an open end of the nasal spray/spout nozzle). In addition, in a form in which two or more nozzles are combined, the entire form including the plurality of nozzles may be regarded as one nozzle and may be simply referred to as "nozzle". As such, the nozzle may be referred to also as a "spout" (or "spout article" /"spout member"), a "member for spraying/spouting a medicament", a "member for spray/spout", or the like.

The term "sub nozzle" as used herein means each nozzle forming an assembly of nozzles in a broad sense, and means each nozzle in a form in which two or more nozzles are combined in a narrow sense. Since the application and function of the "sub nozzle" are approximately the same as the contents described in the definition of the "nozzle", the details thereof are omitted here.

The "tip portion" as used herein means a portion of the tip of each sub nozzle in a broad sense, and means a portion of the tip of the sub nozzle that may be first inserted into a nasal cavity when the sub nozzle is inserted into the nasal cavity in a narrow sense. The "tip portion" is generally a portion that may be inserted into a nasal cavity and thus may have a smaller diameter than at least the external naris.

The term "connected" as used herein refers to joining in a continuous manner, or a state of being joined. The term "connected to each other" means a state in which certain two objects are joined so as to be continuous with each other, but the certain two objects do not necessarily need to be directly connected. For example, when two connected objects are viewed as a whole, it can be said that the objects are "connected to each other" when the objects are in a state of being joined in a continuous manner. Specifically, as described later, even when two objects are "connected" with a "connecting portion" interposed therebetween, it can be said that the objects are "connected to each other" as long as the objects are connected in a continuous manner as a whole. The "connecting portion" as used herein is a portion that is interposed between two objects and contributes to connection. Specifically, it is a portion that indirectly connects two objects to each other.

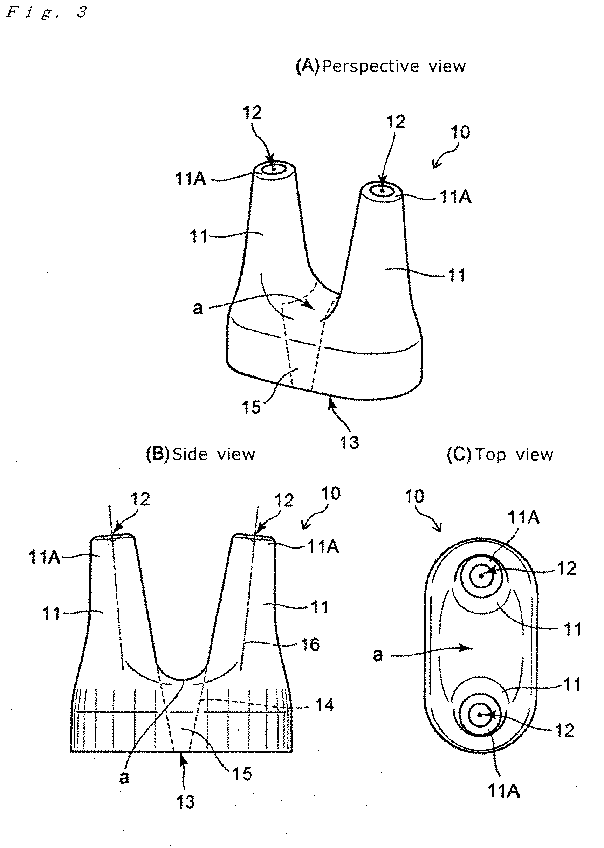

The "axis of the sub nozzle" as used herein refers to an imaginary straight line passing through the central portion of each sub nozzle along a longitudinal direction from the center of the tip portion of the sub nozzle. Specifically, as illustrated in Fig. 3(B) described later, a dashed-dotted line passing through the central portion of each sub nozzle along the longitudinal direction from the center of the tip portion of the sub nozzle is the "axis of the sub nozzle".

The nasal spray/spout nozzle of the present invention is a nozzle article including a pair of sub nozzles capable of discharging a fluid material to the outside. In particular, the nasal spray/spout nozzle of the present invention is a nozzle article capable of spraying/spouting a fluid material represented by a medicament, preferably with a form of spray and/or spout of the medicament. When the nasal spray/spout nozzle of the present invention is used, each of the sub nozzles is inserted into a corresponding one of the two nasal cavities from the tip portion of each of the sub nozzles, and a medicament is sprayed/spouted from the jetting port at the tip portion toward the target site in the nasal cavity. The target site referred to herein is a site to be sprayed/spouted by the nasal spray/spout nozzle.

Features of the present invention will be exemplarily described with reference to Figs. 1(A) to 1(C). Figs. 1(A) to 1(C) exemplarily illustrate a nasal spray/spout nozzle 10. The nasal spray/spout nozzle 10 includes a pair of sub nozzles 11, and each of the sub nozzles 11 is connected with each other in a continuous manner via a connecting portion 15. In the connecting portion 15, a surface portion of a region sandwiched between one sub nozzle and the other sub nozzle is referred to as a crotch portion a. A jetting port 12 is provided at a tip portion 11A of each sub nozzle 11. An end portion axially facing the tip portion 11A having the jetting port 12 is an open end 13. The axis referred to herein is an imaginary straight line that penetrates the central portion of the sub nozzle 11 along a longitudinal direction, and the axis is indicated by a one-dot chain line in Fig. 1(B). The contour shape of the open end 13 includes the contour shape of each sub nozzle 11 and the connecting portion 15.

Each sub nozzle 11 and the connecting portion 15 may be joined to each other and integrated such that the boundary between each sub nozzle 11 and the connecting portion 15 is not visible. As for the boundary, for example, as illustrated in Fig. 1(B), an extension line in an extending direction of the inner side surface of each sub nozzle (that is, the side surface on the side proximal to the crotch portion a) in side view may be a boundary line 14 indicating the boundary between each sub nozzle 11 and the connecting portion 15. Alternatively, the boundary line 14 may be a line that borders a shape in which each of the pair of sub nozzles 11 is congruent with each other as a whole in side view.

Each of the sub nozzles 11 of the nasal spray/spout nozzle 10 may include a hollow portion therein. Such hollow portion is preferably in communication with the jetting port 12. The hollow portion provided in the interior of the nasal spray/spout nozzle 10 may also be in communication with the outside at another nozzle side that is opposed to the side of the nozzle jetting port 12. In other words, the nasal spray/spout nozzle 10 may have its one end as the open end 13, the one end being opposed to the nozzle jetting port 12 in the axial direction of the nasal spray/spout nozzle 10.

As illustrated in Figs. 1(A) to 1(C), in the nasal spray/spout nozzle 10, the separation distance between an axis 16 of one sub nozzle and an axis 16 of the other sub nozzle among the sub nozzles 11 is increased from the connecting portion 15 toward each tip portion 11A. In other words, the axis 16 of one sub nozzle and the axis 16 of the other sub nozzle among the sub nozzles 11 are connected so as to be separated from each other from the connecting portion 15 toward each tip portion 11A. The term "separation distance between the axis 16 of one sub nozzle and the axis 16 of the other sub nozzle among the sub nozzles 11 is increased from the connecting portion 15 toward each tip portion 11A" means at least a form at the time of use. The term "the time of use" as used herein at least refers to a state immediately before the nasal spray/spout nozzle 10 of the present invention is inserted into the nasal cavity and a medicament is sprayed/spouted.

The nasal spray/spout device according to one embodiment of the present invention, having the technical features described above, can more suitably make a medicament reach the target site.

The nasal cavity, which is the inside of a nose, has a tunnel structure with unevenness from the external naris serving as an entrance into the nasal cavity to pharynx (upper pharynx or nasopharynx) forming a part of the throat. Specifically, the inside of the nasal cavity is divided into left and right by a partition called nasal septum located in the middle of the nasal cavity, and the nasal cavity has a series of pleated structures called nasal turbinate at a position in the nasal cavity facing the nasal septum. The nasal turbinate is formed of superior nasal turbinate, middle nasal turbinate, and inferior nasal turbinate. The inside of the nasal cavity generally forms a complex and narrow passage with the nasal septum and the three pleated nasal turbinates.

For example, when a medicament is sprayed/spouted toward a site near the upper pharynx or nasopharynx positioned at the back in the nasal cavity using a nasal spray/spout device, the medicament needs to reach the target site through a complicated and narrow passage in the nasal cavity. When the medicament is sprayed/spouted from the vicinity of the center of the nasal cavity, there is a possibility that the medicament is trapped in the nasal septum, and when the medicament is sprayed/spouted from the vicinity of the outside of the nasal cavity, there is a possibility that the medicament is trapped in an inner wall portion such as a nasal turbinate. That is, when a medicament is sprayed/spouted in such a manner as to avoid a structure in the nasal cavity such as a nasal turbinate and the nasal septum or an inner wall in the nasal cavity, the medicament can efficiently reach the target site.

In the nasal spray/spout nozzle 10 illustrated in Fig. 1, the separation distance between the axis 16 of one sub nozzle and the axis 16 of the other sub nozzle among the sub nozzles 11 is increased from the connecting portion 15 toward each tip portion 11A. As a result, the medicament sprayed/spouted from the jetting port 12 of the tip portion 11A can easily and suitably reach the target site while avoiding the structures in the nasal cavity. Specifically, it is possible to more suitably avoid the medicament from being trapped in the inner wall portion such as the nasal septum and the nasal turbinates. As a result, the medicament delivery to the target site in the nasal cavity is more likely to improve. In other words, the nasal spray/spout nozzle 10 of the present invention can improve the delivery rate of the medicament to the target site.

Such improvement of the medicament delivery is caused by the fact that the medicament can be sprayed/spouted separately from the vicinity of the center of the nasal cavity and the vicinity of the outside of the nasal cavity by increasing the separation distance between the axis 16 of one sub nozzle 11 and the axis 16 of the other sub nozzle 11. This configuration causes the medicament to suitably pass through a complicated and narrow passage in the nasal cavity from the jetting port 12 and reach the target site.

When the nasal spray/spout nozzle 10 as illustrated in Fig. 1 is produced, the sub nozzles 11 may be produced separately, and the sub nozzles 11 may be connected to each other via a connecting portion in another process. Alternatively, the sub nozzle 11 may be produced and connected at the same time using a mold or the like having the shape of the nasal spray/spout nozzle 10, and the sub nozzles 11 may be produced as a molded product to obtain a completed product in a connected state. For example, the sub nozzles may be produced as an integrally molded product of an injection-molded product, or may be produced as an integrally molded product of a three-dimensional object produced by using light beam or the like.

The nasal spray/spout nozzle 10 according to the present invention may be made of a rigid material or a flexible material. Examples of the material of the nasal spray/spout nozzle 10 may include, but not limited to, a resin material, a glass material, a metal material, and a ceramic material. In a preferred embodiment, the nasal spray/spout nozzle 10 may include a resin product (e.g., a resin molded article). The nasal spray/spout nozzle 10 may also include an elastomer or a rubber.

The shape of the sub nozzle 11 of the nasal spray/spout nozzle 10 according to the present invention may take any shape as long as the separation distance between the axis 16 of one sub nozzle and the axis 16 of the other sub nozzle is increased from the connecting portion 15 toward each tip portion 11A. For example, a shape may be taken in which, in side view, the separation distance between the inner side surface of one sub nozzle 11 and the inner side surface of the other sub nozzle 11 is increased from the connecting portion 15 toward each tip portion 11A. Alternatively, a shape may be taken in which the separation distance between the outer side surface of one sub nozzle 11 and the outer side surface of the other sub nozzle 11 is increased, or a shape may be taken in which the separation distance is decreased. Alternatively, a shape may be taken in which the separation distance is constant between the outer side surface of one sub nozzle 11 and the outer side surface of the other sub nozzle 11 being approximately parallel.

For example, a shape may be taken in which the outer side surface of one sub nozzle 11 and the outer side surface of the other sub nozzle 11 are approximately parallel to each other, but the separation distance between the inner side surface of one sub nozzle 11 and the inner side surface of the other sub nozzle 11 is increased from the connecting portion 15 toward each tip portion 11A.

From the viewpoint of facilitating the insertion of the sub nozzle into the nasal cavity, the shape of the sub nozzle 11 may be, in side view, a tapered shape, an approximately conical shape, a cylindrical shape, a frustum shape, or a truncated cone shape, or may be a shape obtained by combining the shapes described above. The shapes described above may be applied to the entire shape of the sub nozzle 11, or may be applied to a part of the shape of the sub nozzle 11.

The sub nozzle may have a bilaterally symmetrical shape and/or a bilaterally asymmetrical shape. For example, in the form illustrated in Fig. 1(B), in side view, the sub nozzle 11 may have a symmetrical shape with respect to the axis 16 of the sub nozzle. In the form illustrated in Fig. 3(B), in side view, the sub nozzle 11 may have a bilaterally symmetrical shape and a bilaterally asymmetrical shape with respect to the axis 16 of the sub nozzle. Specifically, as illustrated in Fig. 3(B), in side view, the tip portion 11A of the sub nozzle has a symmetrical shape with respect to the axis 16 of the sub nozzle, and a portion of the sub nozzle proximal to the crotch portion a has an asymmetrical shape with respect to the axis 16 of the sub nozzle. Since the sub nozzle has a bilaterally symmetrical shape and/or a bilaterally asymmetrical shape, it is easy to insert the sub nozzle into the nasal cavity and to adjust the insertion depth of the sub nozzle into the nasal cavity.

Hereinafter, a possible embodiment of the nasal spray/spout device according to one embodiment of the present invention will be specifically described.

As illustrated in Figs. 1(A) to 1(C), in the nasal spray/spout nozzle 10, the separation distance between the axis 16 of one sub nozzle and the axis 16 of the other sub nozzle 11 among the sub nozzles 11 may be gradually increased from the connecting portion 15 to each tip portion 11A. Alternatively, the distance may be increased in a stepwise manner. Specifically, as illustrated in Fig. 1(B), when a separation distance Lx between the axes 16 of any sub nozzles and a separation distance Ly that is closer to the tip portion 11A side than the separation distance Lx are taken, the separation distance of the axes 16 of the sub nozzles may change in such a manner as to continuously increase from the separation distance Lx to the separation distance Ly.

By having the above-described features, it can be said that the nasal spray/spout nozzle 10 has a portion where the sub nozzle 11 are positioned so as to form an approximately V shape in side view. Specifically, the overall shape of the nasal spray/spout nozzle 10 may be an approximately V shape rather than an approximately U shape. In other words, the term "separation distance between the axis 16 of one sub nozzle 11 and the axis 16 of the other sub nozzle 11 among the sub nozzles 11 is gradually increased" means that each sub nozzle 11 extends in such a manner as to be separated from each other.

The nasal spray/spout nozzle 10 may include at least a portion where the distance between the outer side surface of one sub nozzle 11 and the outer side surface of the other sub nozzle 11 is increased from the connecting portion 15 toward the tip portion 11A in side view. Preferably, a portion where a distance between the outer side surface of one sub nozzle 11 and the outer side surface of the other sub nozzle 11 is partially increased from the connecting portion 15 toward the tip portion 11A may be provided.

In the above-described configuration, when the sub nozzle 11 is inserted into a nasal cavity, the side surface extending outside the sub nozzle 11 can be positioned along the inner wall of the nasal cavity. In other words, the outside of the sub nozzle 11 can be positioned to be able to touch the inner wall of the nasal cavity. As a result, when the user inserts the sub nozzle into the nasal cavity, the position of the sub nozzle 11 in the nasal cavity can be easily grasped by the tactile sensation of the inner wall of the nasal cavity. Thus, the position of the jetting port 12 can be easily grasped, and the spray/spout position of the medicament can be easily adjusted, which can contribute to improvement of the medicament delivery to the target site in the nasal cavity.

The "outer side surface of the sub nozzle" in the present specification means, for example, in Fig. 3(B), the side surface of the sub nozzle 11 on a side distal to the vicinity of the center (or crotch portion a) of the nasal spray/spout nozzle 10 among side surfaces of the sub nozzle 11. In other words, the "outer side surface of the sub nozzle" is the side surface of the sub nozzle 11 on a side not directly facing the side surface of each of the one sub nozzle 11 and the other sub nozzle 11 in side view.

In the nasal spray/spout nozzle 10, the ratio of the longest distance to the shortest distance in the distances between the outer side surface of one sub nozzle 11 and the outer side surface of the other sub nozzle 11 may be larger than 1, and the longest distance corresponds to the distance at the tip. In other words, in the distances between the outer side surface of one sub nozzle 11 and the outer side surface of the other sub nozzle 11, the distance at the tip of the sub nozzle 11 may be longer than the distance of the portion other than the tip of the sub nozzle 11.

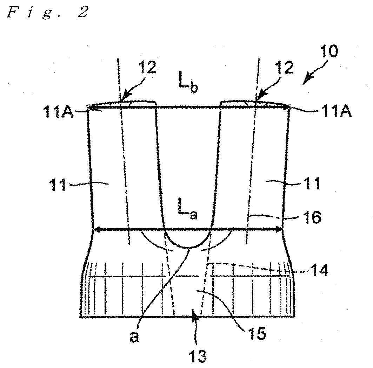

Specifically, as illustrated in Fig. 2, the longest distance L_{b} between the outer side surface of one sub nozzle 11 and the outer side surface of the other sub nozzle 11 is a distance between the tip of one sub nozzle 11 and the tip of the other sub nozzle 11. The ratio of L_{b} to the shortest distance Lₐ between the outer side surface of one sub nozzle 11 and the outer side surface of the other sub nozzle 11 is 1 or more. From the viewpoint of ease of insertion of the sub nozzle 11 into the nasal cavity, the ratio of the longest distance L_{b} to the shortest distance Lₐ may be 1.5 or less, 1.3 or less, 1.2 or less, or 1.1 or less. With the ratio of the longest distance to the shortest distance being within the above numerical range, the position of the sub nozzle inserted into the nasal cavity can be easily grasped from the tactile sensation of the inner wall of the nasal cavity when the user inserts the sub nozzle 11 into the nasal cavity. As a result, the user can easily adjust the sub nozzle to face the target site in the nasal cavity, which can contribute to improvement of medicament delivery.

The separation distance between the axis 16 of one sub nozzle and the axis 16 of the other sub nozzle may be gradually increased from the vicinity of the crotch portion a toward the vicinity of the tip portion 11A as illustrated in Fig. 1(B). In other words, the portion where the separation distance between the axis 16 of one sub nozzle and the axis 16 of the other sub nozzle is gradually increased may be, for example, the entire portion from the vicinity of the crotch portion a to the vicinity of the tip portion 11A as illustrated in Fig. 1(B).

With the above-described structure, the medicament sprayed/spouted from the jetting port 12 of the tip portion 11A can suitably reach the target site while avoiding the structures in the nasal cavity, and the medicament delivery to the target site in the nasal cavity is more likely to improve.

The nasal spray/spout nozzle 10 of the present invention may have a line-symmetric contour shape in top view. Specifically, the contour shape of the nasal spray/spout nozzle 10 may be line-symmetric in a direction orthogonal to a direction forming the pair. The contour shape of the nasal spray/spout nozzle 10 means the shape of a line that forms the periphery of an object to be a target object.

Referring to Fig. 1(C), the contour shape of the nasal spray/spout nozzle in top view surrounds each sub nozzle 11. Further, the contour shape is a line-symmetric contour shape. The line-symmetric contour shape is a contour shape that approximately overlaps the contour shape before reversal when the contour shape is reversed about a certain straight line as an axis on the contour shape of the nasal spray/spout nozzle in top view. Alternatively, it can be said that the line-symmetric contour shape has a line-symmetric contour when the axis is regarded as a crease and one contour-shape portion and the other contour-shape portion having the crease as a boundary can have approximately the same shape.

As illustrated in Fig. 4, in the nasal spray/spout nozzle 10 of the present invention, the separation distance between the axis 16 of one sub nozzle of the sub nozzles 11 and the axis 16 of the other sub nozzle is increased from the connecting portion 15 toward each tip portion 11A. As illustrated in Fig. 4, when the contour shape of the nasal spray/spout nozzle 10 is a line-symmetric shape, the increase in the separation distance between the sub nozzles 11 can be uniform. In other words, the separation distance between the sub nozzles 11 is increased from the connecting portion 15 toward the tip portion 11A may be caused by not only one sub nozzle 11 but also both sub nozzles 11.

Specifically, as illustrated in Fig. 4, the separation distance from the axis 16 of one sub nozzle to the line symmetry axis 17 and the separation distance from the axis 16 of the corresponding other sub nozzle to the line symmetry axis 17 can be approximately equal from the connecting portion 15 toward each tip portion 11A. With the above structure, the directions of the medicaments sprayed/spouted from the sub nozzles can be equal to each other on the left and right. Thus, each medicament sprayed/spouted from both sub nozzles easily reaches the target site in the nasal cavity, and the medicament delivery can further improve.

In one embodiment of the present invention, as illustrated in Fig. 4, the angle formed by the line symmetry axis 17 of the nasal spray/spout nozzle 10 and the axis 16 of the sub nozzle may be an acute angle. The "line symmetry axis of the nasal spray/spout nozzle" as used herein can be said to be an axis that approximately overlaps the nozzle 10 before reversal when the nozzle 10 is reversed about a longitudinal straight line passing over the nasal spray/spout nozzle 10 in side view in Fig. 4. Alternatively, it can be said that the above-described axis is a "line symmetry axis of the nasal spray/spout nozzle" when the axis is regarded as a crease and the portion of one nozzle 10 and the portion of the other nozzle 10 having the crease as a boundary can have approximately the same shape. The line symmetry axis 17 may be a line positioned generally between the sub nozzles 11 and passing from a portion of the crotch a through the open end 13.

When it is assumed that a vertical direction in Fig. 4 (that is, the longitudinal direction of the nasal spray/spout nozzle) is the direction in which gravity acts, the angle formed by the vertical axis and the axis 16 of the sub nozzle may be an acute angle. Alternatively, in Fig. 4, it can be said that the axis 16 of the sub nozzle is inclined such that the axis 16 of the sub nozzle forms an acute angle with respect to the vertical axis (longitudinal axis at the nozzle center).

In the exemplary embodiment illustrated in Fig 4, the axis 16 of each sub nozzle is at an angle θ with respect to the line symmetry axis 17 of the nasal spray/spout nozzle. In other words, when it is assumed that the vertical direction of the axis 16 of the sub nozzle is the direction in which gravity acts, the axis 16 of the sub nozzle is inclined so as to form the angle θ with respect to the vertical axis. As illustrated in Fig. 4, the angle θ is an acute angle.

When the angle formed by the line symmetry axis 17 of the nasal spray/spout nozzle 10 and the axis 16 of the sub nozzle is an acute angle, the medicament sprayed/spouted from the jetting port 12 of the sub nozzle 11 inserted into the nasal cavity is more likely to reach the target site. In addition, the sub nozzle 11 is easily inserted into the nasal cavity, and hardly comes into contact with the inner wall of the nasal cavity or the like, and thus the user is less likely to feel discomfort.

Here, the angle θ formed by the axis 16 of the sub nozzle and the line symmetry axis 17 of the nasal spray/spout nozzle 10 may be appropriately adjusted according to the target site where the medicament to be sprayed/spouted reaches as long as it is an acute angle. For example, the angle θ may be 1° or more from the viewpoint of positioning the jetting port 12 of the sub nozzle 11 at a position distal from the nasal septum when the sub nozzle 11 is inserted into the nasal cavity. Alternatively, the angle may be 2° or more, 3° or more, 4° or more, or 5° or more. From the viewpoint of positioning the jetting port 12 at a position distal from the nasal turbinate or the like, the angle θ may be 15° or less, and may be 12° or less, 10° or less, 8° or less, 7° or less, or 6° or less. As an example, the angle θ may be from 2° to 15°.

When the angle θ is within the above range, it is easy to avoid the sprayed/spouted medicament from being trapped in the inner wall of the nasal cavity. As a result, the medicament delivery to the target site in the nasal cavity is more likely to improve.

In one embodiment of the present invention, the connecting portion 15 may be positioned on an inner side surface of each of the sub nozzles 11. In other words, the connecting portion 15 may be positioned between the side surface of one sub nozzle 11 and the side surface of the other sub nozzle. Here, the "inner side surface of the sub nozzle" means a side surface of the sub nozzle 11 that is on the proximal side with respect to the vicinity of the center of the nasal spray/spout nozzle 10 in Fig. 3(B). In other words, it means a side surface of the sub nozzle 11 on the proximal side of the crotch portion a.

Further, in one embodiment of the present invention, the connecting portion 15 may be provided on the inner side surface on the proximal side from the open end 13 among the inner side surfaces of the sub nozzle 11. In other words, the connecting portion 15 may be provided on the inner side surface of the sub nozzle on the distal side from the jetting port 12. The connecting portion 15 may form the open end 13 integrally with each sub nozzle 11 at the open end 13. By adopting the above-described form, when the pair of sub nozzles 11 are inserted into the nasal cavity, the connecting portion 15 is provided at a position capable of abutting on the nasal columella.

With the above structure, when the nasal spray/spout nozzle 10 is inserted into the nasal cavity, the connecting portion 15 easily abuts on the nasal columella. That is, when the nasal spray/spout nozzle 10 is inserted into the nasal cavity and the connecting portion 15 and the nasal columella come into contact with each other, the nasal spray/spout nozzle 10 can be positioned at the position at the time of contact. This makes it easy to make the insertion depth of the sub nozzle 11 constant. In other words, the insertion position of the nozzle jetting port 12 in the nasal cavity can be more appropriately determined by abutting at least a part of the connecting portion 15 on the nasal columella. That is, the length of the nasal spray/spout nozzle 10 to be inserted into the nasal cavity can be easily fixed in such a positional relationship that the medicament can more suitably pass through a complicated and narrow passage in the nasal cavity. As a result, the medicament delivery is more likely to improve. Further, since the insertion position of the sub nozzle 11 can be more appropriately determined by abutting the connecting portion 15 on the nasal columella, the effect that can be exhibited by the portion where the separation distance between the axes 16 of the sub nozzles is increased, which is a feature of the present invention, can be further improved.

The term "abutting" as used herein refers to a contacting with a pressing mode. Examples of the "abutting" as used herein include at least one selected from the group consisting of "contacting with a push mode", "contacting with a slide and subsequent stop mode", "contacting with a hook mode", and "contacting with an locking mode".

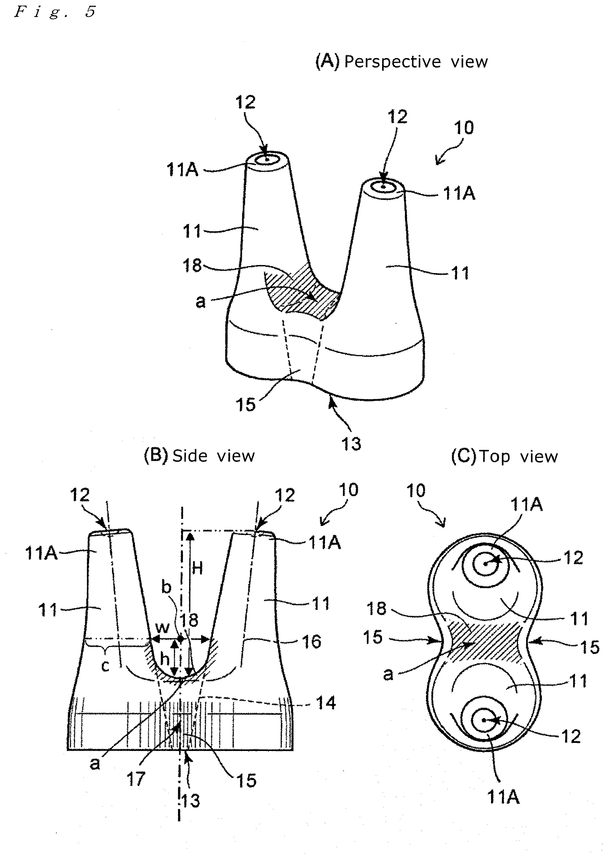

In one embodiment of the present invention, the connecting portion 15 and a part of each of the sub nozzles 11 may form a pressing surface 18 for pressing the nasal columella. Specifically, as illustrated in Figs. 5(A) to 5(C), the crotch portion a of the connecting portion 15 of the nasal spray/spout nozzle 10, the inner side surface of each sub nozzle 11, and the surface connecting the crotch portion a and the inner side surface of each sub nozzle 11 may be integrated to form a "pressing surface for pressing the nasal columella".

In other words, the pressing surface 18 may be a surface extending from the crotch portion a of the connecting portion 15 to the inner side surface of each sub nozzle 11. For example, the pressing surface 18 may be a surface extending from the crotch portion a to a part of the inner side surface of each sub nozzle 11 until the width between the inner side surfaces of the sub nozzles 11 becomes a width w. As illustrated in Fig. 5(B), the width w means a distance from the inner side surface of one sub nozzle 11 to the inner side surface of the other sub nozzle 11 on an axis perpendicular to the line symmetry axis 17 of the nasal spray/spout nozzle 10. The width w indicates the width of the pressing surface 18 extending from the crotch portion a to a part of the inner side surface of each sub nozzle 11.

The width w of the pressing surface 18 is not particularly limited as long as the pressing surface 18 can press the nasal columella. For example, the width w may be approximately the same as the width W of the nasal columella. Being approximately the same as the width W of the nasal columella means that, for example, the width w of the pressing surface 18 is 0.9W to 1.1W with respect to the width W of the nasal columella. In another aspect, the width w of the pressing surface 18 may be larger or smaller than the width W of the nasal columella.

From the viewpoint of bringing the pressing surface 18 into closer contact with the nasal columella when the pressing surface 18 presses the nasal columella, the width w of the pressing surface 18 may be 100% or less, preferably 95% or less, more preferably 90% or less, and still more preferably 85% or less with respect to the width W of the nasal columella.

From the viewpoint of increasing the region where the pressing surface 18 can press the nasal columella, the width w of the pressing surface 18 may be 40% or more, preferably 50% or more, more preferably 60% or more, and still more preferably 70% or more with respect to the width W of the nasal columella. Since the nasal columella is generally flexible, it can be easily deformed by external forces. Thus, when the sub nozzle 11 is inserted into the nasal cavity, the nasal columella is deformed so as to have a width approximately equal to the width w even when the width w of the pressing surface 18 is smaller than the width W of the nasal columella. In other words, compression is performed such that the width W of the nasal columella is approximately equal to the width w of the pressing surface 18.

The pressing surface 18 may be a surface extending from the crotch portion a to a height h from the crotch a to a part of the inner side surface of each sub nozzle 11. As illustrated in Fig. 5(B), the height h means a distance from the crotch portion a to a point b away from the crotch portion a by a predetermined distance in a direction proximal to the jetting port 12 on the line symmetry axis 17 of the nasal spray/spout nozzle 10. The height h and the distance between a and b indicate the height of the pressing surface 18 extending from the crotch portion a to a part of the inner side surface of each sub nozzle 11. The height h of the pressing surface 18 is not particularly limited as long as the pressing surface 18 can press the nasal columella.

The term "to press the nasal columella" means that the user intentionally or purposefully presses the nasal columella, and does not mean that the user unconsciously or accidentally presses the nasal columella. Specifically, the "pressing surface for pressing the nasal columella" is a surface used by the user to intentionally press the nasal columella in order to achieve the effects described below when the sub nozzle 11 is inserted into the nasal cavity.

With the above structure, when the sub nozzle 11 of the nasal spray/spout nozzle 10 is inserted into the nasal cavity, the sub nozzle 11 is easily inserted into the nasal cavity to such an extent that the pressing surface 18 presses the nasal columella. When the pressing surface 18 presses the nasal columella with such a force that the nasal columella deforms, the nasal spray/spout nozzle 10 is supported by the nasal columella, and as a result, the insertion position and the insertion depth of the sub nozzle 11 inserted into the nasal cavity are easily fixed. Since the insertion position and the insertion depth are fixed, the direction of the medicament to be sprayed/spouted from the tip portion 11A of the sub nozzle 11 can be easily controlled, and the medicament is likely to reach the target site.

In one embodiment of the present invention, the pressing surface 18 may be a surface that can press the nasal columella from both sides of the nasal columella with the nasal columella interposed therebetween. With this configuration, when the sub nozzle 11 is inserted into the nasal cavity, the nasal columella is sandwiched between the pressing surfaces 18, and both sides of the nasal columella are pressed. In other words, the pressing surface 18 can press so as to hold the nasal columella. Alternatively, the pressing surface 18 may be a close contact surface that constricts the nasal columella from the left and right.

The term "the pressing surface 18 can press the nasal columella from both sides of the nasal columella" refers to a state in which the crotch portion a of the nasal spray/spout nozzle 10 presses the nasal columella and each of the inner side surfaces of the sub nozzles 11 press the nasal columella or both sides of the nasal columella and the nasal septum. That is, it can be said that the entire peripheral portion of the nasal columella is pressed by the pressing surface 18.

In a preferred aspect, the width w of the pressing surface 18 pressing the nasal columella from both sides of the nasal columella may be approximately the same as the width W of the nasal columella. The width w may be at least approximately the same as the width W of the nasal columella or smaller than the width W of the nasal columella from the viewpoint of enabling the pressing surface 18 to sandwich and press more stably the nasal columella from both sides of the nasal columella. Specifically, the width w may be 100% or less, preferably 95% or less, more preferably 90% or less, and still more preferably 85% or less with respect to the width W of the nasal columella. From the viewpoint of increasing the region where the pressing surface 18 can press the nasal columella, the width w may be 40% or more, preferably 50% or more, more preferably 60% or more, and still more preferably 70% or more with respect to the width W of the nasal columella.

In another aspect, as illustrated in Fig. 5(B), the width w of the pressing surface 18 may be smaller than a sub nozzle diameter c on an extension direction of the axis indicating the width w among the axes perpendicular to the line symmetry axis 17. Specifically, when the width w of the pressing surface 18 and the sub nozzle diameter c are compared at the same height level, the width w of the pressing surface 18 may be 5/6 or less of the sub nozzle diameter c, or may be half or less of the sub nozzle diameter c.

From the viewpoint of bringing the pressing surface 18 into closer contact with the nasal columella when the pressing surface 18 presses the nasal columella, the width w may be less than 100% of the sub nozzle diameter c, and may be preferably 95% or less, more preferably 90% or less, still more preferably 85% or less, and particularly preferably 80% or less. From the viewpoint of increasing the region where the pressing surface 18 can press the nasal columella, the width w may be a width larger than 5% of the sub nozzle diameter c, and may be a width of preferably 10% or more, more preferably 15% or more, still more preferably 20% or more, and particularly preferably 40% or more of the sub nozzle diameter c.

The height h of the pressing surface 18 sandwiching and pressing the nasal columella from both sides of the nasal columella may be any height as long as the pressing surface 18 can sandwich and press the nasal columella from both sides of the nasal columella. For example, the height h may be such a height that only the nasal columella can be pressed from both sides thereof. Alternatively, not only the nasal columella but also the nasal septum and/or the portion connecting the nasal columella and the nasal septum may be pressed from both sides thereof.

From the viewpoint of enabling the pressing surface 18 to sandwich and press more stably at least the nasal columella from both sides of the nasal columella, the height h may be 10% or more, preferably 20% or more, more preferably 30% or more, still more preferably 50% or more, and particularly preferably 70% or more with respect to the width W of the nasal columella. From the viewpoint of reducing discomfort when the sub nozzle 11 is inserted into the nasal cavity, the height h may be 200% or less, preferably 175% or less, more preferably 150% or less, still more preferably 125%, and particularly preferably 100% or less with respect to the width W of the nasal columella.

In another aspect, as illustrated in Fig. 5(B), the height h of the pressing surface 18 may be smaller than the vertical height H from the crotch portion a to the jetting port 12 on the line symmetry axis 17, for example. Specifically, the height h of the pressing surface 18 may be less than or equal to half of the vertical height H, or may be less than or equal to 1/4.

From the viewpoint of enabling the pressing surface 18 to sandwich and press more stably at least the nasal columella from both sides of the nasal columella, the height h of the pressing surface 18 may be at least 1% or more, preferably 5% or more, more preferably 10% or more, and still more preferably 15% or more of the vertical height H from the crotch portion a to the jetting port 12, for example. From the viewpoint of reducing discomfort when the sub nozzle 11 is inserted into the nasal cavity, the height h of the pressing surface 18 may be at least 50% or less of the vertical height H, and may be preferably 40% or less, more preferably 30% or less, and still more preferably 25% or less, for example.

According to such a form, the sub nozzle 11 can be more closely attached to the peripheral portion of the nasal columella or the nasal columella and the nasal septum and further supported, and the insertion position and the insertion depth of the sub nozzle 11 inserted into the nasal cavity can be more stably fixed. In particular, it becomes easy to stably fix the positional relationship in the left-right direction (for example, the direction of the nasal alar side) when the nozzle is inserted into the nasal cavity, and the medicament is more likely to reach the target site.

The force of the pressing surface 18 sandwiching and "pressing the nasal columella from both sides" can be adjusted by changing the separation distance between the inner side surfaces of the sub nozzles 11 of the nasal spray/spout nozzle. For example, by increasing the separation distance between the inner side surfaces of the sub nozzles 11, the force of the pressing surface 18 "pressing the nasal columella from both sides" when the nozzle is inserted into the nasal cavity becomes relatively small, and by decreasing the separation distance, the force of the pressing surface 18 "pressing the nasal columella from both sides" when the nozzle is inserted into the nasal cavity becomes relatively large.

The force of the pressing surface 18 sandwiching and "pressing the nasal columella from both sides" is not particularly limited, and may be appropriately determined according to the size of the nasal columella and/or the size of the nasal cavity of the user of the nasal spray/spout nozzle. When the pressing surface 18 can "press the nasal columella and/or the nasal septum from both sides" with the sub nozzle 11 being inserted into the nasal cavity, the pressing surface 18 can exhibit a function such as a clip, and it becomes easy to fix the spraying/spouting direction of the medicament and to stably determine the insertion position and the insertion depth of the sub nozzle. In this regard, the pressing surface 18 in one embodiment can be said to be a clamping portion capable of clamping at least the nasal columella. In a more preferred aspect, the pressing surface 18 can be said to be a clamping portion capable of clamping the nasal columella and the nasal septum. For example, the pressing surface 18 can also be referred to as a clip portion or a clip-like clamping portion.

When the pressing surface 18 presses the nasal columella, the pressing surface may press other parts of the nose simultaneously with the nasal columella. Alternatively, the pressing surface 18 may be used as the pressing surface 18 for pressing a part of the nose other than the nasal columella. Specifically, the pressing surface 18 may press the nasal cavity that is the inner side of the nose, the outer nose that is the outer side of the nose, a site located around the nose, and a site of the nose connecting a site of the nose and another site of the nose.

Specifically, as a site of the nose, the pressing surface 18 may press a part of the outer nose around the external naris. More specifically, the nasal apex, the nasal alar, the nasal vestibule, the both sides of the philtrum, and the philtrum may be pressed. In addition, the pressing surface 18 may press the nasal septum, the nasal cavity side portion of the nasal alar, and the nasal cavity side portion of the nasal apex in the nasal cavity. Since the pressing surface 18 presses the part of the nose together with the nasal columella, it becomes easy to fix the spraying/spouting direction of the medicament, and the medicament delivery to the target site in the nasal cavity can further improve.

The shape of the pressing surface 18 is not particularly limited as long as it has a function of pressing the nasal columella or pressing the nasal columella from both sides in such a manner as to sandwich the nasal columella. Specifically, the shape of the pressing surface 18 is not particularly limited as long as the connecting portion 15 and a part of each of the sub nozzles 11 are in close contact with the nasal columella at least in series when the crotch portion a abuts the nasal columella with the sub nozzle 11 being inserted into the nasal cavity.

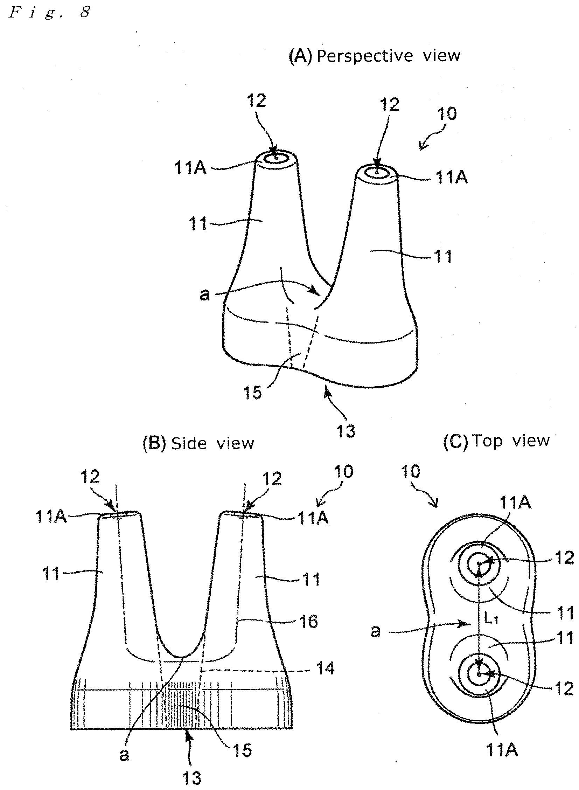

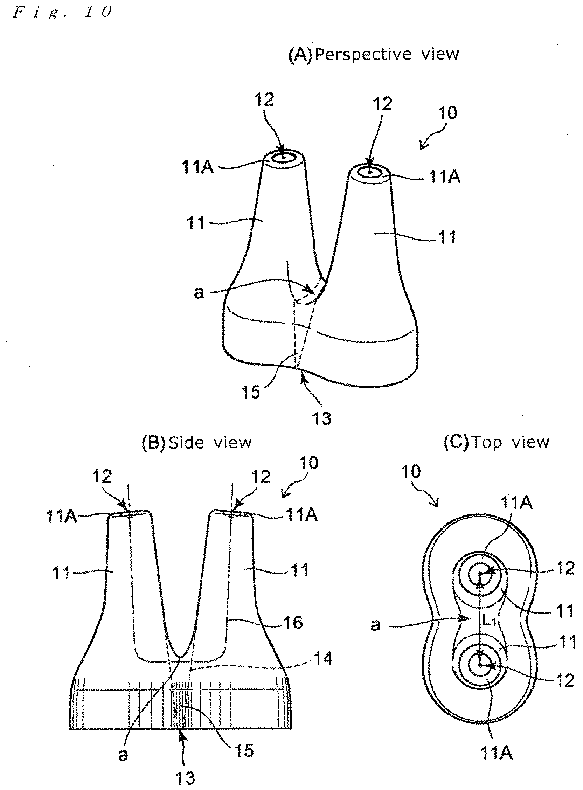

For example, as illustrated in Fig. 8(B), the pressing surface 18 may be formed in an approximately U shape or a rounded shape around the crotch portion a by a part of the connecting portion 15 and each sub nozzle 11. The pressing surface 18 may have a curved surface and/or a flat surface, and may have a bent portion. In Fig. 10(B), the pressing surface 18 is relatively narrower than that in Fig. 3(B), and has a substantial V shape. In any form, the above effect of the pressing surface 18 can be achieved.

In one embodiment of the present invention, as illustrated in Fig. 3(B), the pressing surface 18 may be formed of a curved surface in side view. The radius of curvature of the curved surface of the pressing surface 18 may be, for example, a radius of curvature (unit mm) of 0.1 R or more and 10.0 R or less, 0.5 R or more and 8 R or less, 1 R or more and 5R or less, 1.5 R or more and 4 R or less, or 2.0 R or more and 3 R or less when the center of curvature is placed on the line symmetry axis 17 of the nasal spray/spout nozzle. When the radius of curvature of the pressing surface 18 is within the above range, the pressing surface 18 and the nasal columella can be more easily brought into close contact with each other. Thus, the positional relationship in the left-right direction when the nozzle is inserted into the nasal cavity is more stably fixed, and the medicament is more likely to reach the target site.

In one embodiment of the present invention, in the top view of Fig. 5(C), regarding the nozzle bottom direction in a direction orthogonal to the direction forming the pair, the nozzle bottom dimension in the connecting portion 15 may be smaller than the nozzle bottom dimension in the sub nozzle 11. The direction forming the pair may be, for example, an extending direction of a virtual line connecting the tip portions 11A of the pair of sub nozzles or the jetting ports 12. The nozzle bottom is a portion of the nasal spray/spout nozzle 10 on the open end 13 side at a position facing the tip portion 11A of the nasal spray/spout nozzle 10. Specifically, the nozzle bottom is a portion including each sub nozzle 11 located in the vicinity of the open end 13 and the connecting portion 15. In other words, when the nasal spray/spout nozzle 10 is viewed as a whole, the nozzle bottom can also be said to be a base of the nasal spray/spout nozzle 10 from which each sub nozzle 11 protrudes.

Further, it can be said that the nozzle bottom contour shape of the nasal spray/spout nozzle 10 as illustrated in Fig. 5(C) is narrowed at the connecting portion 15. The nozzle bottom contour shape means the "contour shape" of the "nozzle bottom" defined above. The fact that the nozzle bottom contour shape is narrowed at the connecting portion 15 in top view means, for example, that the nozzle contour shape at the connecting portion 15 is narrowed in such a manner as to shrink. In other words, in top view, the nozzle bottom contour shape of one connecting portion 15 and the nozzle bottom contour shape of the other connecting portion 15 of the nozzle bottom contour shapes of the connecting portions 15 are narrowed so as to approach each other.

The nasal spray/spout nozzle 10 according to one embodiment of the present invention is used by inserting each of the sub nozzles 11 into the nasal cavities and spraying/spouting a medicament toward a target site. At this time, the direction of spraying/spouting the medicament can be appropriately adjusted depending on the target site. For example, when the medicament is sprayed/spouted to a portion in the nasal cavity distal to the external naris, such as the upper pharynx or nasopharynx, a direction and an angle in which the medicament is to be sprayed/spouted are adjusted such that each of the sub nozzles 11 inserted into the nasal cavity is lifted upward (direction of nasal apex).

When the sub nozzle 11 is lifted in a state of being inserted into the nasal cavity, a part of the connecting portion 15, for example, a side surface of the connecting portion 15 abuts on a peripheral portion of the external naris such as the nasal columella or the nasal apex. In this regard, when the nozzle bottom contour shape is narrowed at the connecting portion 15, the peripheral portion of the external naris can be brought into close contact with the nozzle bottom contour shape along the narrowed portion. That is, the narrowed portion of the nozzle bottom dimension and the peripheral portion of the external naris can be locked to each other. In this regard, it can be said that the narrowed portion of the nozzle bottom dimension is a locking portion that can be locked to a portion around the external naris. When the nozzle bottom contour shape is narrowed at the connecting portion 15, the contact area between at least a part of the side surface of the connecting portion 15 and the peripheral portion of the external naris can be further increased at the time of use. Thus, the nasal spray/spout nozzle can be pressed so as to be stably aligned with the peripheral portion of the external naris. As a result, it becomes easier to more suitably determine the position of the jetting port 12 in the nasal cavity, which contributes to improvement of medicament delivery. In this respect, it can be said that the nozzle bottom includes a second pressing surface for pressing the peripheral portion of the external naris.

As illustrated in Figs. 5(A) and 5(C), the nozzle bottom dimension of the connecting portion is smaller than the nozzle bottom dimension of the sub nozzle 11. The form in which the nozzle bottom dimension at the connecting portion is small is not particularly limited.

For example, in Fig. 5(C), the nozzle bottom dimension of the connecting portion is formed into a curved surface. More specifically, the nozzle bottom dimension in the connecting portion forms a recess so as to have a smooth curved surface. In other words, a rounded recess is formed.

When the nozzle bottom dimension has a curved surface, the nasal spray/spout nozzle 10 is lifted in a state where the sub nozzle 11 is inserted into the nasal cavity, and the connecting portion 15 abuts on the peripheral portion of the external naris, the connecting portion 15 and the peripheral portion of the external naris can be more closely attached. This is because the curved surface of the nozzle bottom dimension at the connecting portion functions as a recess and comes in close contact to receive, as a protrusion, a portion such as the nasal apex, the nasal columella, and/or the nose alar in the peripheral portion of the external naris. As a result, the nasal spray/spout nozzle 10 can be more stably pressed against the peripheral portion of the external naris. As a result, the position of the jetting port 12 in the nasal cavity can be determined in a more suitable manner, which contributes to improvement of medicament delivery.

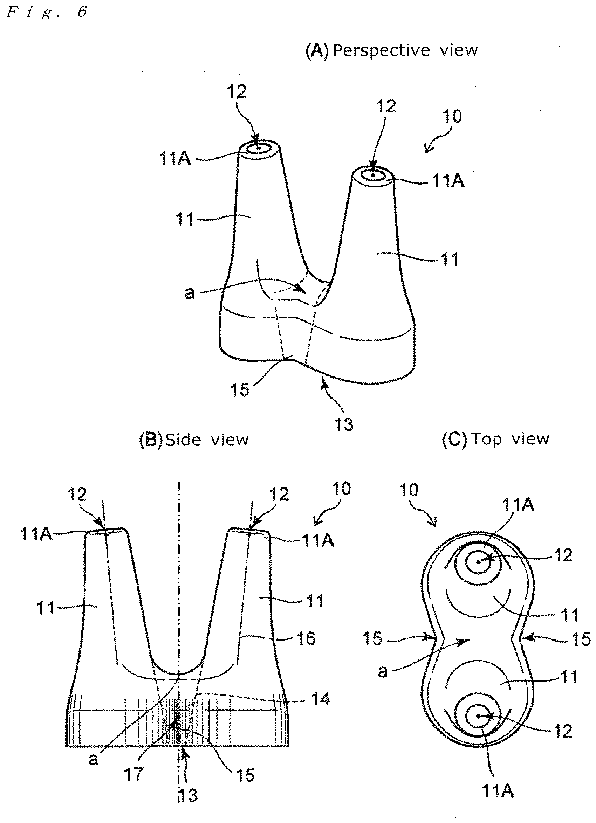

As illustrated in Figs. 6(A) and 6(C), the nozzle bottom dimension at the connecting portion 15 of the nose spray/spout nozzle according to one embodiment may form a recess so as to form a corner. In other words, the nozzle bottom dimension of the connecting portion 15 may be reduced so as to form an approximately V shape. While the nozzle bottom dimension of the connecting portion 15 in the aspects of Figs. 5(A) and 5(C) is formed into a curved recess, the nozzle bottom dimension of the connecting portion 15 in the aspects of Figs. 6(A) and 6(C) is not formed into a curved recess.

With such a configuration, when the nasal spray/spout nozzle 10 is lifted in a state where the sub nozzle 11 is inserted into the nasal cavity, and the connecting portion 15 forming the recess in such a manner as to form a substantial V shape abuts on the peripheral portion of the external naris, the nasal spray/spout nozzle 10 can be more stably fixed to the peripheral portion of the external naris. Specifically, since the two approximately V-shaped surfaces are in contact with each other so as to press both sides of the peripheral portion of the external naris, the nasal spray/spout nozzle 10 can be more stably fixed. The above feature contributes to improvement of the medicament delivery of the nozzle of the present invention.

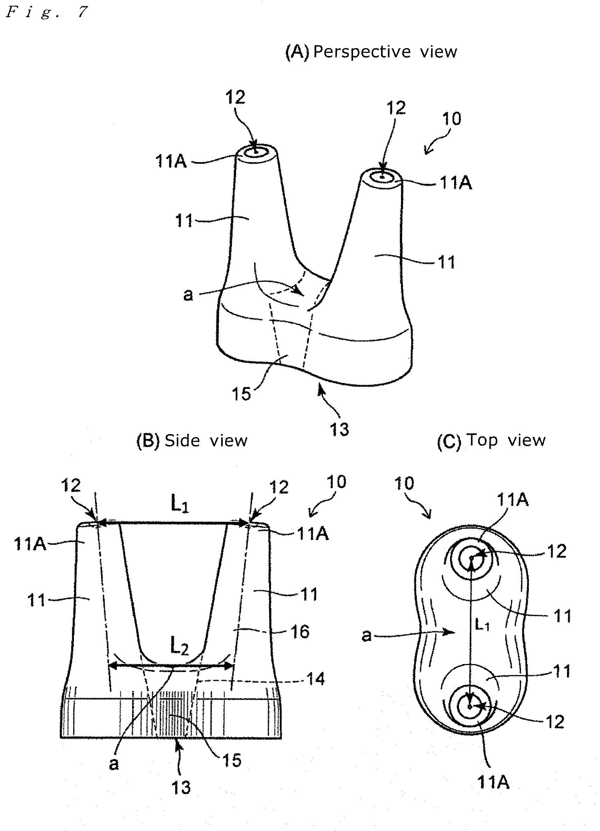

The distance between the sub nozzles 11 of the nasal spray/spout nozzle 10 may be appropriately adjusted according to the size, shape, and the like of the nasal cavity of the user and/or the target site. Specifically, as illustrated in Figs. 7(B) and 7(C), a separation distance Li between the axes of both the sub nozzles in the jetting port 12 of one sub nozzle 11 and the jetting port 12 of the other sub nozzle 11 may be appropriately adjusted according to the size, shape, and the like of the external naris of the user.

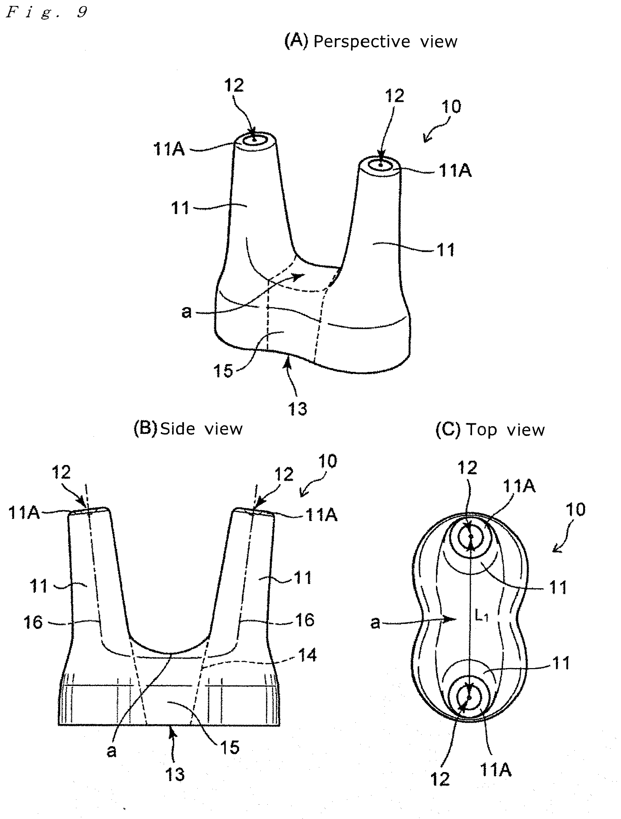

For example, as illustrated in Fig. 8(C), the separation distance Li may be shorter than that in the form illustrated in Fig. 7, or the separation distance Li may be further shortened as illustrated in Fig. 10. Such a form can be suitable for use for infants and children whose nasal cavity is relatively smaller than that of adults. On the other hand, as illustrated in Fig. 9, the separation distance Li may be longer than that in the form illustrated in Fig. 7. Such a form can be suitable for use for a patient or the like having a relatively large nasal cavity. Further, a separation distance L₂ between the axes of both the sub nozzles at the point where the line symmetry axis 17 of the nasal spray/spout nozzle 10 and the crotch portion a intersect may be adjusted for the same reason as for the separation distance Li.

As illustrated in Figs. 7(B) and 7(C), the ratio L₁/L₂ between the separation distance Li and the separation distance L₂ may be larger than 1.0. L₁/L₂ may be 1.5 or less, and may be 1.3 or less and 1.1 or less. When L₁/L₂ is larger than 1.0, the axes of both the sub nozzles are easily positioned in a direction away from each other. Thus, the jetting port 12 when the sub nozzle 11 is inserted into the nasal cavity is easily positioned in the direction distal from the nasal septum, and the medicament is less likely to be trapped in the nasal septum. When L₁/L₂ is 1.5 or less, the jetting port 12 is easily positioned in the direction distal from the nasal turbinate, and thus, the medicament is less likely to be trapped in the nasal turbinates and the like. As a result, the medicament delivery to the target site is likely to improve.

To obtain an aspect having L₁/L₂ in the above range, the separation distance Li may be 5 mm or more and 30 mm or less, or may be 5 mm or more and 20 mm or less. The separation distance L₂ may be 5 mm or more and 30 mm or less, and may be 5 mm or more and 20 mm or less.

Each of the sub nozzles 11 may gradually decrease in diameter from the connecting portion 15 toward the tip portion 11A. The fact that each of the sub nozzles 11 gradually decreases in diameter means that the barrel dimension of the sub nozzle gradually decreases. The sub nozzle 11 may gradually decrease in diameter toward the jetting port 12, or may decrease in a stepwise manner. The shape of the sub nozzle 11 that gradually decreases in diameter may be, for example, a tapered shape, and may take, for example, a conical shape, a frustum shape, or a truncated cone shape.

In the above configuration, when the nasal spray/spout nozzle 10 is inserted into the nasal cavity, it can be made possible for the portion where the sub nozzle reduces in size to more suitably fit the wall surface of the internal naris. That is, the nasal spray/spout nozzle 10 can be stably pressed against the wall surface of the internal naris by the portion of the sub nozzle 11 reduced in diameter, and the insertion position and direction of the nozzle jetting port 12 can be particularly suitably determined. In addition, it is possible to mitigate an undesirable impact generated on the wall surface of the internal naris upon inserting the nasal spray/spout nozzle 10 into the nasal cavity, and thereby making it easier to reduce the discomfort such as a pain generated in a user (e.g., a patient).

The nasal spray/spout nozzle 10 of the present invention may have rigidity, and the shape may be maintained constant before and after use. Alternatively, the nasal spray/spout nozzle 10 may have flexibility, and the shape may change during use to become the nasal spray/spout nozzle 10 of the present invention. That is, deflection may occur through insertion of the flexible nasal spray/spout nozzle 10 into the nasal cavity, and the shape thereof may change.

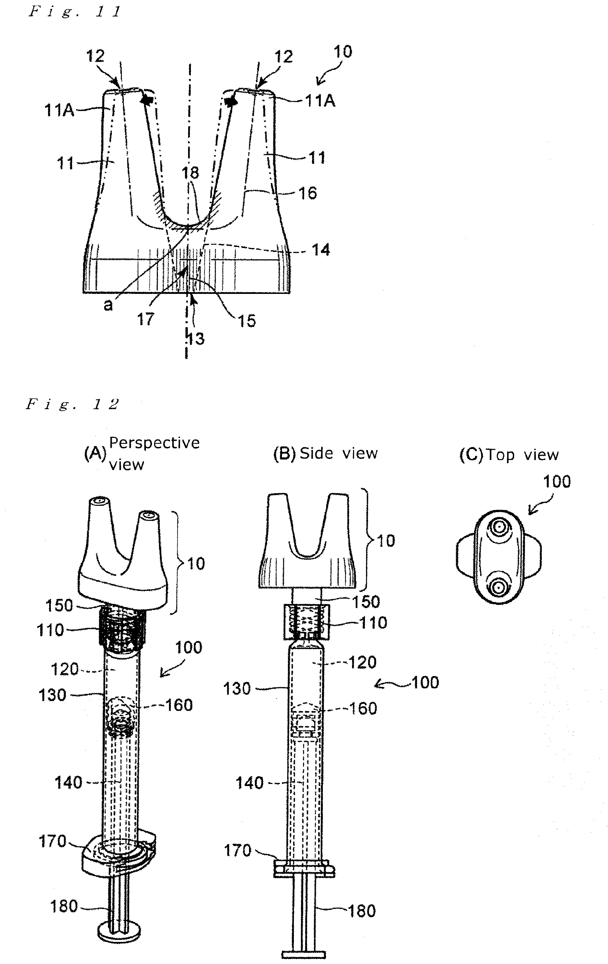

For example, as illustrated in Fig. 11, when the nasal spray/spout nozzle 10 is inserted into the nasal cavity, the sub nozzles 11 may be spread apart from each other. When the nasal spray/spout nozzle 10 is inserted into the nasal cavity, the pressing surface 18 presses the nasal columella. The crotch portion a and the inner side surface of each sub nozzle 11 constituting the pressing surface 18 receive a reaction force from the nasal columella being pressed. Since the reaction force from the nasal columella acts in a direction to laterally push and spread the inner surface of each sub nozzle 11, when the sub nozzle has flexibility, the axis of the sub nozzle may be displaced so as to spread outward (directions of the arrows in Fig. 11). The shape in which "the separation distance between the axis of one sub nozzles and the axis of the other sub nozzle is increased from the connecting portion toward the tip" may be achieved by this configuration. When the shape of the nasal spray/spout nozzle 10 is displaceable during use as described above, the nasal spray/spout nozzle 10 can be easily inserted into the nasal cavity. From the above features, it can be said that the nasal spray/spout nozzle 10 in one embodiment includes a flexible sub nozzle. In other words, it can be said that the nasal spray/spout nozzle 10 in one embodiment includes a sub nozzle whose shape is displaceable during use.

When the nasal spray/spout nozzle 10 has flexibility, the nasal spray/spout nozzle 10 may include, for example, a flexible material, and specifically, may include, for example, an elastomer or a rubber-like substance. For example, silicone rubber, fluororubber, butadiene rubber, isoprene rubber, and/or nitrile rubber may be used as the material of the nasal spray/spout nozzle 10.

### (Spray/Spout Device)

Examples of a nasal spray/spout device using the nasal spray/spout nozzle 10 may include, for example, a conventional spray/spout device, and also an upper exhaust airless-type spray/spout device and a syringe-type spray/spout device. Such nasal spray/spout device can include the nasal spray/spout nozzle incorporated thereinto. Here, the syringe-type spray/spout device will be described in detail.

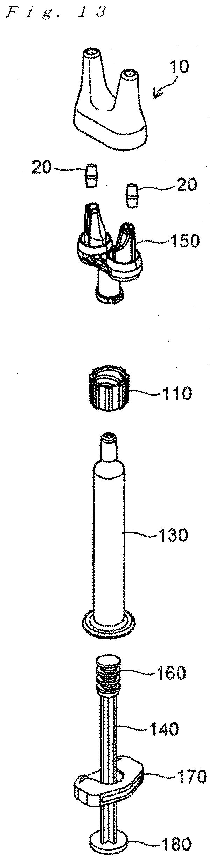

Fig. 12 illustrates a syringe-type spray/spout device 100 including the nasal spray/spout nozzle 10. Fig. 13 illustrates, as an exploded perspective view, the syringe-type spray/spout device 100 including the nasal spray/spout nozzle 10. The syringe-type spray/spout device 100 includes the nasal spray/spout nozzle 10, a syringe body 130 including a syringe barrel 120, a plunger rod 140 inserted into the syringe barrel 120 of the syringe body 130, a piston 160 provided at an end of the plunger rod 140 on the proximal side of the nozzle 10, and a finger rest 170 disposed around an end of the syringe body 130 on the distal side of the nozzle 10.

More specifically, as illustrated in Fig. 13, the syringe-type spray/spout device 100 includes the syringe body 130 made of a synthetic resin or glass and having the syringe barrel 120 capable of being filled with a medicament, the plunger rod 140 inserted into the syringe barrel 120 of the syringe body 130, the piston 160 configured to slide in the syringe barrel 120 to deliver the medicament in the syringe barrel 120 from the syringe body 130 to the jetting port 12 of the tip portion 11A of the nozzle 10, a finger rest 170 disposed around an end of the syringe body 130 distal side of the nozzle 10 that transmits a force applied from a finger of a practitioner such as a doctor to the plunger rod 140, and a plunger 180.

In a broad sense, the term "finger rest" as used herein refers to a portion for putting a finger thereon during use of the nasal spray/spout nozzle 10. In a narrow sense, the term "finger rest" as used herein refers to a portion for putting a finger when the nasal spray/spout nozzle 10 is inserted into the nasal cavity. From this viewpoint, the finger rest 170 may also be referred to as a "finger hook", a "finger grip" or the like.

As illustrated in Fig. 13, the nasal spray/spout nozzle 10 is connected to the syringe body 130 via an adapter 150, and the adapter 150 and the syringe body 130 are fixed by a screw tip 110. The adapter 150 may be inserted from the open end 13 side of the nasal spray/spout nozzle 10. Thus, the adapter 150 has a shape similar to that of the nasal spray/spout nozzle 10, and its size is smaller than that of the nasal spray/spout nozzle 10. In the adapter 150, a rod 20 is provided in each of adapter 150 portions corresponding to the sub nozzles 11 at the time of inserting the nasal spray/spout nozzle 10.

### (Method of Using Spray/Spout Device)

The user can use the spray/spout device 100 in accordance with the following procedures, for example.
(1) Two fingers (for example, an index finger and a middle finger) are disposed at positions on the syringe body 130 side of the finger rest 170, and a thumb is disposed at an endmost portion of the plunger 180.
(2) The nasal spray/spout nozzle 10 is oriented such that the disposed two fingers can form approximately a parallel relationship with the longitudinal direction (or the width direction) of the nasal columella.
(3) The tip portion 11A of the sub nozzle 11 of the nasal spray/spout nozzle 10 is inserted into the nasal cavity. Here, the tip portion 11A is inserted to such an extent that the crotch portion a is pressed against the peripheral portion of the external naris (for example, the nasal columella).
(4) The plunger 180 is pushed toward the side proximal to the nasal spray/spout nozzle 10, whereby the medicament is sprayed/spouted into the nasal cavity.

In the nasal spray/spout device 100 using the nasal spray/spout nozzle 10 of the present invention, the medicament delivery to the target site of the nasal cavity can be further improved by the effect exhibited by the nasal spray/spout nozzle 10 of the present invention. In addition, the nasal spray/spout device 100 according to the present invention slides the plunger 180 in such a manner as to push the plunger 180 against the syringe barrel 120 filled with a medicament, thereby sending the medicament from the syringe barrel 120 to the jetting port 12. Since the nasal spray/spout nozzle 10 according to the present invention includes the pair of sub nozzles 11, the jetting ports also form a pair. In this regard, as compared with a conventional nasal spray/spout nozzle having only a single jetting port 12, the amount of spraying/spouting the medicament is increased, and thus the force required for sliding the plunger 180 can be reduced. In addition, since there are two jetting ports 12, the force for sliding the plunger 180 to spray/spout the medicament can be relatively reduced as compared with a conventional nozzle having only a single jetting port.

Although some embodiments of the present invention have been hereinbefore described, they are regarded for illustrative purpose regarding the typical ones. The present invention is not limited to these embodiments. It would be readily appreciated by those skilled in the art that various embodiments are possible. For example, the internal structure of the nasal spray/spout nozzle may be the same as the internal structure of a conventional nozzle.

### INDUSTRIAL APPLICABILITY

The nasal spray/spout nozzle 10 according to the present invention can have a more suitable delivery efficiency of a medicament with respect to the target site having a complex intranasal structure. Specifically, the separation distance between the axis 16 of one sub nozzle and the axis 16 of the other sub nozzle is increased from the connecting portion toward the tips. Thus, it is possible to realize a nasal spray/spout type medical device capable of delivering a medical to a wide range of a target site and obtaining higher medicament efficacy.

### EXPLANATION OF REFERENCES

- 10: Nasal spray/spout nozzle
- 11: Sub nozzle
- 11A: Tip portion
- 12: Jetting port
- 13: Open end
- 14: Boundary line
- 15: Connecting portion
- 16: Axis of sub nozzle
- 17: Line symmetry axis of nose spray/spout nozzle
- 18: Pressing surface
- H: Vertical height from crotch portion a to jetting port 12
- c: Sub nozzle diameter in width direction of pressing surface
- w: Width of pressing surface
- h: Height of pressing surface
- a: Crotch portion
- 20: Rod
- 100: Syringe-type spray/spout device
- 110: Screw tip
- 120: Syringe barrel
- 130: Syringe body
- 140: Plunger rod
- 150: Adapter
- 160: Piston
- 170: Finger rest
- 180: Plunger

## Claims

1. A nasal spray/spout nozzle comprising a pair of sub nozzles, wherein
a tip of each of the sub nozzles is provided with a jetting port, and the sub nozzles are connected to each other via a connecting portion, and
a separation distance between an axis of one of the sub nozzles and an axis of the other sub nozzle is increased from the connecting portion toward the tip.

2. The nasal spray/spout nozzle according to claim 1, wherein the separation distance is gradually increased.

3. The nasal spray/spout nozzle according to claim 1 or 2, wherein an angle formed by a line symmetry axis of the nasal spray/spout nozzle and the axis of the sub nozzle is an acute angle in side view.

4. The nasal spray/spout nozzle according to claim 3, wherein the acute angle is from 2° to 15°.

5. The nasal spray/spout nozzle according to any one of claims 1 to 4, wherein a contour shape of the nasal spray/spout nozzle is a line-symmetric shape in top view.

6. The nasal spray/spout nozzle according to any one of claims 1 to 5, wherein the connecting portion is positioned on an inner side surface of each of the sub nozzles.

7. The nasal spray/spout nozzle according to any one of claims 1 to 6, wherein the connecting portion and a portion of each of the sub nozzles form a pressing surface for pressing a nasal columella.

8. The nasal spray/spout nozzle according to claim 7, wherein the pressing surface is a surface that can sandwich and press the nasal columella from both sides of the nasal columella.

9. The nasal spray/spout nozzle according to any one of claims 1 to 8, wherein a nozzle bottom dimension of the connecting portion, the nozzle bottom dimension being in a direction orthogonal to a direction forming the pair in top view, is smaller than a nozzle bottom dimension of the sub nozzle, the nozzle bottom dimension being in the direction orthogonal to the direction forming the pair in top view.

10. The nasal spray/spout nozzle according to any one of claims 1 to 9, wherein a nozzle bottom contour shape is narrowed at the connecting portion in top view.

11. The nasal spray/spout nozzle according to any one of claims 1 to 10, wherein each of the sub nozzles is gradually decreased in diameter from the connecting portion toward the tip.

12. The nasal spray/spout nozzle according to any one of claims 1 to 11, further comprising at least a portion where a distance between an outer side surface of the one sub nozzle and an outer side surface of the other sub nozzle is increased from the connecting portion toward the tip in side view.

13. The nasal spray/spout nozzle according to any one of claims 1 to 12, wherein a ratio of a longest distance to a shortest distance between an outer side surface of the one sub nozzle and an outer side surface of the other sub nozzle is more than 1, and the longest distance corresponds to the distance at the tip.

14. A nasal spray/spout device comprising the nasal spray/spout nozzle according to any one of claims 1 to 13.
